(19)

Europäisches
Patentamt
European
Patent Office
Office européen
des brevets

(11) **EP 2 623 139 A1**

(12) **EUROPEAN PATENT APPLICATION**

(43) Date of publication:
**07.08.2013 Bulletin 2013/32**

(51) Int Cl.:
*A61M 1/28* (2006.01)

(21) Application number: **13152920.8**

(22) Date of filing: **28.01.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **01.02.2012 IT MO20120021**

(71) Applicants:
• **La Milia, Vincenzo**
  **23849 Rogeno (LC) (IT)**

• **Grandi, Fabio**
  **47100 Forli (FC) (IT)**

(72) Inventors:
• **La Milia, Vincenzo**
  **23849 Rogeno (LC) (IT)**
• **Grandi, Fabio**
  **47100 Forli (FC) (IT)**

(74) Representative: **Villanova, Massimo et al**
  **Luppi Crugnola & Partners S.r.l.**
  **Viale Corassori, 54**
  **41124 Modena (IT)**

(54) **Peritoneal dialysis system**

(57)    A peritoneal dialysis system is disclosed in which a conductimeter (5), arranged on a discharge line (2) of a peritoneal dialysis circuit, measures the electrical conductivity of the liquid introduced into the peritoneal cavity, with variable dwell times. The conductivity values are used in a mathematical model to determine the operation of the peritoneal membrane and other parameters relating to the peritoneal dialysis.

Fig. 1

EP 2 623 139 A1

**Description**

**Background of the invention**

The invention relates to a system for peritoneal dialysis.

Specifically, but not exclusively, the invention can be used to determine, during peritoneal dialysis, a treatment parameter, for example the functionality of the peritoneal membrane.

Other fields of application of the invention can be: 1) measuring the concentration of glucose in the solutions used for peritoneal dialysis; 2) measuring the concentration of sodium in the solutions used for peritoneal dialysis; 3) measuring residual volume, after partial or complete drainage of the peritoneal cavity; measuring residual volume is particularly useful in the case of a malfunction of the peritoneal catheter or during certain types of automated peritoneal dialysis treatment, such as, for example tidal treatment; 4) early diagnosis of peritonitis during automated peritoneal treatment (APD); 5) monitoring peritonitis and response to antibiotic treatment.

In order to assess the function of the peritoneal membrane, tests of the functionality of the peritoneal membrane are now necessary, such as the so-called peritoneal equilibration test (PET), which, nevertheless, even in the simplest forms thereof, is always complicated and requires a significant expenditure of time and resources (running tests, laboratory dosing, data interpretation, etc).

**Summary of the invention**

One of the objects of the invention is to provide a system that is able to determine one or more parameters relating to treatment during peritoneal dialysis.

One advantage of the invention is to determine, simply and reliably, parameters such as the functionality of the peritoneal membrane (in particular the conveying capacity thereof), the residual volume, the glucose and/or sodium concentration in the solutions used for the peritoneal dialysis, early diagnosis and monitoring of peritonitis, etc.

One of the other objects of the invention is to devise a method that is implementable with the aforesaid system.

During peritoneal dialysis, especially with a peritoneal solution that is very hypertonic with respect to the blood plasma (for example with a 3.86% glucose solution), in the first part of the exchange, especially during the first hour, a marked reduction of the concentration of sodium in the solution occurs.

This effect is due, as demonstrated by the so-called "three pore theory", to the conveying of free water, devoid of solutes, that occurs through the aquaporin-1 channels or micropores, of the peritoneal membrane.

Further, in the first part of an exchange with the aforesaid methods, conveying free water may constitute even 50% of the ultrafiltration. This conveying free water dilutes the sodium and all the other electrolytes in the solution in the peritoneal cavity, even if the most marked effect, because of the high concentrations of sodium in solutions for peritoneal dialysis, is related to the sodium.

In the second part of an exchange, there is, on the other hand, an increase in the concentration of sodium in the peritoneal solution, due to the marked reduction of the conveying of free water, and to the spread of sodium from the blood plasma to the solution.

It is demonstrated that the reduction sodium concentration in the first part of the exchange (with hypertonic solution) indicates good operation of the channels of the aquaporin-1. It has in fact been found that in optimum membrane conditions, this concentration reduction, in the first part of the exchange (for example in the first hour), can be higher than 10 mmol/L, whereas in normal membrane operating conditions this reduction is between 5 mmol/L and 10 mmol/L; in conditions of reduced conveying of free water, this concentration reduction can be less than 5 mmol/L.

In the solutions for peritoneal dialysis high glucose concentrations are also present (from 1.36% to 3.86%, i.e. from 1360 mg/dl to 3860 mg/dl, whereas the normal plasma concentration varies from 80 to 110 mg/dl). During a peritoneal exchange the glucose is absorbed by the blood capillaries in the peritoneum; the speed of absorption depends on the functionality of the peritoneal membrane; in fact, patients with rapid peritoneal transport ("rapid conveyer" patients) will have higher glucose absorption speed than patients with slow conveying of the peritoneal membrane ("slow conveyer" patients). These glucose absorption features are exploited during the PET to classify the patients undergoing peritoneal dialysis.

The conductivity of a solution also depends on the glucose concentration, which increases the viscosity of the solution and reduces electrical conductivity. Thus the measurement of the variation in conductivity in the solution that is introduced into the peritoneal cavity, between the start of an exchange and after a certain period of time, will also be the expression of the glucose absorption and thus of the function of the peritoneal membrane.

In conformity to these premises, as the electrical conductivity of a solution is a function of the electrolytic solution and of the glucose, it is deemed to be useful to assess the variations in the conductivity of the dialysis solution during peritoneal dialysis treatment, in particular with a hypertonic dialysis liquid (for example a solution with 3.86% glucose) in order to assess the operation of the peritoneal membrane in a simple and rapid manner (instantaneously).

During this treatment, during the first part of the exchange, there will be a dilution of the electrolytes in the peritoneal solution, especially of the sodium, which is, as known, the electrolyte that most contributes to electrical conductivity. Further, there will also be an absorption of glucose and the consequent variation in the concentration will also cause a variation in electrical conductivity.

The variation in electrical conductivity, during a peritoneal exchange with hypertonic solution, will thus be simultaneously an expression of the conveying of free water (that dilutes the sodium concentration and reduces electrical conductivity) and of the conveying of the small solutes, like glucose, the reabsorption of which will cause a reduction in the viscosity of the solution and thus an increase in electrical conductivity.

The degree of variation of the electrical conductivity of the solution during a peritoneal exchange will thus be an expression of the functionality of the peritoneal membrane in the patients undergoing dialysis, and can permit the study thereof and of the variations thereof over time, in a simple and rapid manner.

The conductivity measurement could be used to run both CAPD (continuous ambulatory peritoneal dialysis or manual dialysis) and APD (automated peritoneal dialysis) to test peritoneal functionality.

The peritoneal functionality test, based on variations of electrical conductivity, can be run without any need for blood samples and without need for laboratory dosages both in the blood and in the solution used to make the peritoneal dialysis.

For example, the method in question could comprise a test conducted with a fresh peritoneal solution with a 3.86% glucose concentration, the conductivity of which may be measured during the line washing manoeuvres; after a certain peritoneal dwell period (for example after an hour) it suffices to drain a part (just a few ml are enough) of the peritoneal liquid and determine the electrical conductivity thereof. The variation in conductivity, at the first hour of peritoneal dwelling, it will above all express the conveying of free water and thus of operation of the channels of the aquaporin-1 of the peritoneal membrane; in other words, if the electrical conductivity increases little (or even remains the same or decreases) this will indicate good operation of the channels of the aquaporin-1 of the peritoneal membrane; if on the other hand a significant increase of conductivity occurs, again at the first hour, this could be an expression of reduced operation of the channels of the aquaporin-1 of the peritoneal membrane.

If, after 4 hours of dwelling of the solution with a 3.86% glucose solution, the measurement of electrical conductivity is repeated (always by draining just a few ml of solution), the variation in conductivity, between the start of the test and after 4 hours, will be the expression of the conveying of small solutes (in this case the solute of interest is glucose) and this change in conductivity can enable the type of conveying of the peritoneal membrane, of every single patient to be classified in a similar manner to PET, but with a method that is simple and can be read immediately.

Conveying features of the peritoneal membrane are used both for prescribing better peritoneal dialysis methods for the patient and for monitoring the peritoneal membrane over time, in order to prevent certain worrying complications such as encapsulating peritoneal sclerosis.

For simplicity of measurements, the method in question could comprise a test conducted at the patient's home and could be repeated several times (for example once a month).

A digital conductimeter, with sufficient memory that is applied to the peritoneal dialysis circuit (for example to the discharge line of the circuit) could enable the measured data to be stored, which can be analysed subsequently in the dialysis centre or be transmitted in real time electronically.

In one example the method in question could comprise the combination of the measurement of conductivity with the measurement of another parameter, for example the volume of peritoneal solution introduced into the peritoneal cavity of the patient and/or the volume of solution drained from the peritoneal cavity. This second measurement could be performed, for example, via a flowmeter (in particular an external flowmeter that is not in contact with the peritoneal solution) or by means of weighing scale.

In one example, the method in question could comprise a test used on various types of peritoneal solution to assess, simply and rapidly, the influence of the type of different peritoneal solutions on conveying the peritoneal membrane.

In one example, the method in question could be used for measuring the sodium concentration in an automated peritoneal dialysis method that takes account of the variation in the sodium concentration during treatment by using solutions with the same glucose concentration and different sodium concentrations. Measuring the sodium concentration could be very useful for conducting customised peritoneal dialyses on the basis of patient needs ("modulated" APD), for example a sodium concentration could be used in the higher solution when high glucose concentrations are used (in which sodium is above all removed by convection) and a lower sodium concentration when solutions with a lower glucose concentration are used such as to enable diffuse removal of sodium, for example in patients with hypertension.

In one example, the method in question could be used to measure the glucose concentration in an automated peritoneal dialysis method that takes account of the variation of the glucose concentration during treatment using solutions with the same sodium concentration and different glucose concentrations. Measuring the glucose concentration could be very useful to perform customised peritoneal dialyses on the basis of patients' needs, for example solutions with a higher glucose concentration could be used during the first part of an automated peritoneal dialysis session and the solutions with a lower glucose concentration during the second part of the session ("modulated" APD).

In one example, measuring conductivity during "flush before fill" could also be used in CAPD to evaluate the correctness of the glucose concentration of the fresh peritoneal solution to be used, in particular when multicompartmental bags are used in which it is possible to have errors in mixing the solutions in the different compartments. Measuring conductivity could thus be a safety factor.

In one example, an external conductimeter could be used (applied, for example, to the discharge line of the peritoneal dialysis circuit) that does not require direct contact with the peritoneal solution.

In one example, the method in question could be used to measure the size of the residual volume (VR), by measuring the conductivity of the solution that was already in the peritoneal cavity, the conductivity of the fresh solution that is introduced into the peritoneal cavity after the preceding one has been drained, and the conductivity of the solution that has just been introduced into the peritoneal cavity by draining a small amount (just a few ml) immediately after the end of the infusion of the fresh solution. Measuring the VR can be very useful in the case of suspected dislocation or malfunction of the peritoneal catheter, instantaneously obtaining the size of the VR.

In order to evaluate the VR, the volume (Vinf) of infused solution is measured (by means of a flow meter or bag weighing means), the conductivity (Cd1) of the peritoneal solution used in the discharge step of the preceding cycle, the conductivity (Cd2) of the fresh peritoneal solution (for example measured with an external conductimeter or during the "flush before fill" manoeuvre), and the conductivity (Cd3) of the peritoneal solution used immediately after the end of the infusion of the fresh solution; the conductivity Cd3 could be measured by discharging a quantity of solution, immediately after the end of the infusion, releasing about 50 ml of liquid to eliminate the liquid of the dead space.

To calculate VR, the following formula can be used:

$$VR \text{ (ml)} = Vinf \text{ (ml)} * (Cd3 - Cd2) / (Cd1 - Cd3)$$

For example, with an infusion volume Vinf = 2000 ml, conductivity of the preceding cycle discharge Cd1 = 12.642 mS/cm, conductivity of the fresh solution Cd2 = 12.186 mS/cm, conductivity after infusion Cd3 = 12.300 mS/cm, there will be VR=667 ml ("normal" VR is 200-250 ml).

In one example, measuring the VR, determined with conductivity measurements as stated above, could be further used in APD, for example by a conductimeter inserted into the APD machine (for example in the discharge line). The APD machine may have a sensor (for example a flowmeter or weighing means) to measure or calculate, at each charge, the size of the intraperitoneal volume and of the residual volume. In one example, a monitor of an APD machine could be configured to perform a tidal peritoneal dialysis (TPD) determining the residual volume and varying the volume of peritoneal solution used to be unloaded in function of the determined VR, in order to optimise the TPD, avoiding risks of peritoneal dialysis overfill or underfill, without the need to perform complete discharges.

In one example, the method in question could comprise, during APD treatment, for example during every single treatment break, a step of removing (for example by discharging a few ml of used peritoneal solution) quantities of solution and measuring the conductivity thereof. These conductivity values can then be used to evaluate the suitable moment in which to perform discharging (a sort of "break-point adequacy").

With these values, in fact, it is possible to have information on the glucose and sodium concentration of the peritoneal solution in the peritoneal space and/or ultrafiltration could be estimated; in particular, by means of these values it will be possible to estimate the reabsorption of glucose and/or the conveying of the small solutes (using in particular the known correlation between the peritoneal permeability values, expressed by the known D/PCreat parameter and the electrical conductivity values measured during treatment).

In one example, the method in question could comprise using the measure of conductivity for early diagnosis of peritonitis during treatment with APD, wherein for great volumes it is difficult to become aware of the cloudiness of the discharged liquid that is one of the signs of peritonitis.

It is known that during acute peritoneal phlogosis that occurs during peritonitis glucose absorption increases and there is greater conveying of small solutes like urea, creatinine, sodium, etc. These variations in the conveying of solutes will necessarily be translated into variations in conductivity, above all during the first dwell steps of the solution in the peritoneal cavity. Such conductivity variations are difficult to show after very long dwell periods, for example of about eight hours, as occurs in CAPD, as after very long dwell periods of the solution in the peritoneal cavity, the conductivity in the peritoneal solution is normally the highest that can be obtained.

On the other hand, the aforesaid conductivity variation in the solution could be greater, and more easily measurable, during exchanges an APD treatment, as in this case the average duration thereof is much less, for example about an hour. During APD treatment this enables early diagnoses of peritonitis to be made. It is in fact known that in APD it is difficult to suspect peritonitis by observing the clearness of the peritoneal solution used, as occurs in CAPD, because the solution used is discharged directly into the sewers, or is collected in large bags or containers, where there is a certain bacterial proliferation that makes the liquid cloudy even in the absence of peritonitis.

In one example, the method in question comprises the step of performing measuring conductivity during APD treatment at each discharge; a possible sudden or gradual increase of electrical conductivity could be indicative of the appearance of peritonitis, with the possibility of counting white cells and a culture examination and treating in advance this feared complication of peritoneal dialysis.

In one example, the method in question comprises inserting the conductimeter on the discharge line of an apparatus for APD that measures conductivity at each discharge, alerting by an alarm to sudden increases in conductivity compared with the basic values of the patient.

In one example, measuring conductivity could also be used to evaluate the progress of the phlogosis process during peritonitis. In particular, the method in question could comprise the step of draining a portion of used peritoneal solution after a short dwell period in the peritoneal space (for example after 60 minutes). This can be repeated to evaluate the evolution of conductivity and thus the evolution of the peritonitis.

In one example, the system in question comprises an electrical conductivity sensor arranged in (a discharge line of) a peritoneal dialysis circuit. The sensor may measure, in particular, the electrical conductivity of two used peritoneal solutions that differ from one another by the fact of having stayed in the peritoneal space for different dwell periods. The measured values are used in a mathematical model to determine at least one peritoneal dialysis parameter, for example the functionality of the peritoneal membrane.

Such objects and advantages, and still others, are achieved by the method and the system according to one or more of the claims said out below.

## Brief description of the drawings

The invention can be better understood and implemented with reference to the attached drawings that illustrate an embodiment thereof by way of non-limiting example.

Figure 1 is a diagram of a system of peritoneal dialysis according to the invention.

Figure 2 shows an embodiment of a peritoneal dialysis system according to the invention.

Figure 3 shows another embodiment of the peritoneal dialysis system.

Figure 4 is a diagram of the system in figure 3 provided with a user interface screen connected to the system measuring unit.

Figure 5 is a diagram of the control logic unit of the system in figure 4.

Figure 6 is a time chart of a first manner of controlling the system in figure 4.

Figure 7 is a time chart of a second manner of controlling the system in figure 4.

Figure 8 is a time chart of a third manner of controlling the system in figure 4.

## Detailed description

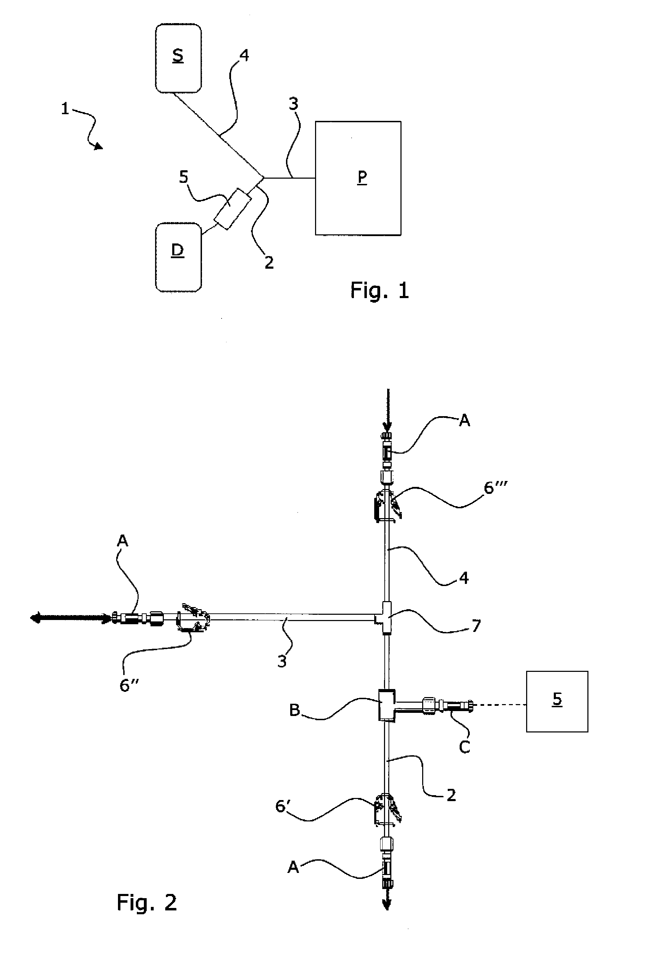

With reference to the aforesaid figure 1, with 1 overall a peritoneal dialysis system (PD) has been indicated. The PD system comprises, in particular, an extracorporeal circuit for peritoneal dialysis that will be connectable with a peritoneal access (for example a peritoneal catheter) of a patient P for conveying a liquid (for example used peritoneal solution) coming from the peritoneal space or cavity and/or a liquid (for example fresh peritoneal solution) intended for filling the peritoneal space or cavity.

The extracorporeal circuit may comprise, as in the case in point, a discharge line 2, and access line 3 and a delivery line 4, for example joined together in a three-way joining zone. The discharge line 2 is connectable to a discharge D (for example a discharge bag or a hydraulic discharge network). The access line 3 is configured for connecting a peritoneal access of the patient P to the discharge line 2. The delivery line 4 is configured for connecting a source S of fresh peritoneal solution (for example a supply bag or an on-line preparing device) to the discharge line 2 and to the access line 3.

The peritoneal dialysis system 1 comprises a measuring unit 5 arranged in the extracorporeal circuit for detecting at least one value of electrical conductivity of the liquid in the circuit. The measuring unit 5 may be arranged, as in this case, to operate in the discharge line 2. The measuring unit 5 may comprise, for example, a submersible conductimeter (arranged submerged in the discharge line 2), or a conductimeter without contact (arranged around the discharge line 2). It is also possible to use (for example in a set for CAPD) a conductimeter inserted into the discharge line, which will be divided into two portions, each of which will have an end connected, respectively, to an input and an output of the conductimeter. The conductimeter could also be arranged on a branch line connected to the discharge line 2. It is possible to provide a direct connection of one end of the discharge line (for example to an APD machine) to a conductimeter input.

Further on in the description, similar elements, common to the various embodiments, will be indicated by the same numbering for the sake of simplicity.

In figure 2, the peritoneal dialysis system comprises, in the specific case, a disposable set for CAPD.

The PD system comprises means for selectively closing the discharge line 2, the access line 3 and the delivery line 4. Such closing means may comprise, as in the case of figure 1, three (manually drivable) grippers or clamps 6', 6", 6"', each arranged, respectively, on the discharge line 2, on the access line 3, on the delivery line 4.

The discharge line 2, the access line 3 e the delivery line 4 may be, for example, joined together by a three-way joint zone 7. Each line 2, 3 and 4 will end with a connector A of known type, suitable for (removably) connecting, respectively, to the used liquid discharge D, the fresh liquid source S and the access to the peritoneal space of the patient P.

The measuring unit 5 may be connected to the discharge line 2, for example, by means of a connector C (removable connection) that is in turn connected as branch to the line 2. In particular, the connector C may communicate with a measuring basin B inserted into the discharge line 2.

Each PD system disclosed here may comprise an evaluation and calculation unit for determining at least one peritoneal dialysis parameter on the basis of at least one electrical conductivity value of the liquid found in the extracorporeal circuit. The evaluation and calculation unit may be connected to the measuring unit 5. The evaluation and calculation unit may comprise a microprocessor, for example built into the measuring unit 5, and/or a microcomputer connected (in a known manner, also electronically and/or by wireless) to the measuring unit 5.

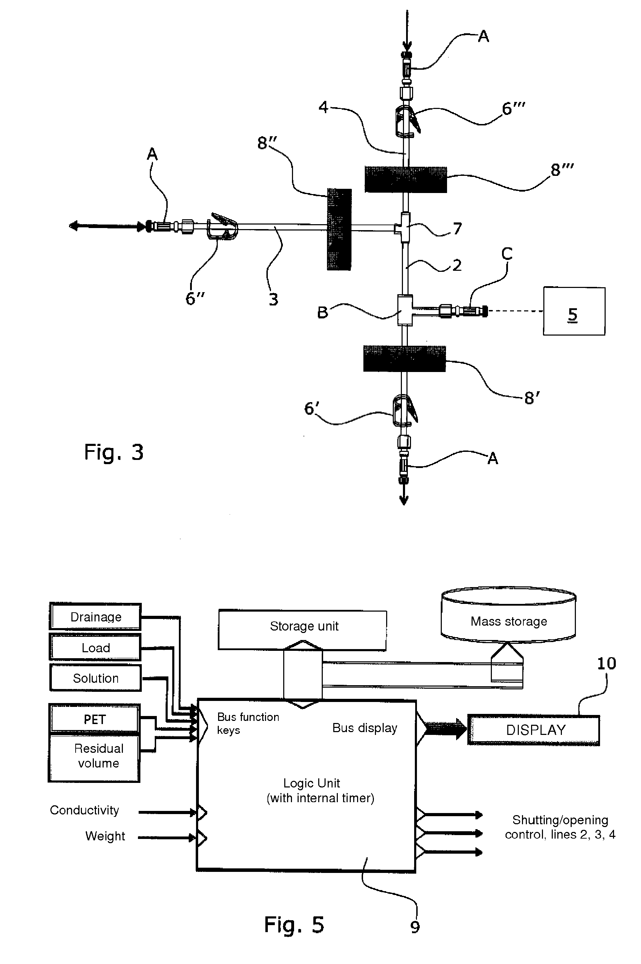

In the system illustrated in figure 3, the means for selectively closing the discharge line 2, the access line 3 and the delivery line 4, comprise three automatically driven closing devices 8', 8", 8"', for example three electrically driven grippers.

The closing devices 8', 8", 8"' may be connected to a programmable electronic control unit 9 (figure 5) that may be connected to the measuring unit 7 and to the evaluation and calculation unit.

The electronic control unit 9 may be configured for controlling the means for closing the lines of the circuit.

The PD system may further comprise a medium that is readable by computer and bears programme instructions that are runnable by the control unit. This medium may be a removable or non-removable support.

The PD system may comprise a user interface, for example a graphic interface, connected to the control unit 9. The user interface may comprise a screen 10.

The control unit 9 may be further connected to a weighing device (for example one or more scales) of the bags, and/or to means (for example flowmeters) for measuring the flow of the liquid supplied to the peritoneal space and discharged from the space.

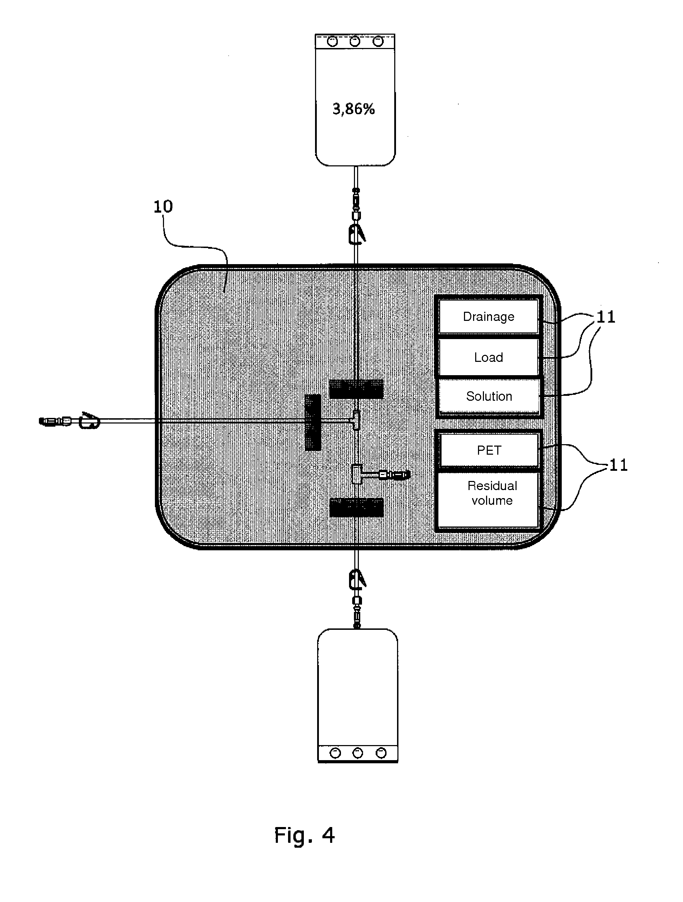

With reference to the diagram in figure 4, wherein the source of the fresh dialysis liquid comprises a supply bag and the discharge of the used dialysis liquid comprises a discharge bag, the control unit 9 may be programmed to show on the screen 10 of the user interface function keys 11 to enable the operator to command the various system functions.

The control unit 9 will have a timer for performing the various operations within the scheduled times. In figure 5, the control unit 9 has an inlet for receiving the conductivity signals from the measuring unit 5, and may have an input for receiving the weight signals of the bags. The control unit 9 will have the outputs of the control signals of the closing devices of the circuit lines. The control unit 9 may further receive the commands from the user interface: for example a command (by the "Drain" button) for performing the discharge step, and/or a command ("Load" button) for performing the filling step, and/or a command ("Solution" button) for acquiring the conductivity of the fresh peritoneal solution, and/or a command ("PET" button) for running the functionality test of the peritoneal membrane, and/or a command ("Residual volume" button) to run the algorithm determining the VR.

The measuring unit 5 may comprise, by way of example, a conductivity sensor or probe, with the following technical features: resolution = 5 mS/cm; accuracy = $\pm 1$ % of the full scale reading; response time = 5 sec at 98% of the full scale reading, 15 sec at 100% of the full scale reading; measurement range = 1 $\mu$S/cm - 200 mS/cm; temperature compensation = automatic compensation between 20° C and 38° C; operating temperature range = between 5° C and 85°C; dimensions = 4 mm sensor diameter; housing = up to the immersion point indicated on the probe.

The sensor means of the weight/volume of the liquid entering/exiting the peritoneal space may comprise an least one pair of scales with the following technical features: resolution = 5 g; measurement range = max. capacity 5 kg; housing = hooked.

A specific example of the various operating steps (1 to 8) of the method for determining a peritoneal dialysis parameter during treatment is given below.

First example:

1. Discharging a quantity (for example 50 g) of used peritoneal solution coming from the peritoneal space (for example after the nocturnal dwell time or after at least 2 or 3 hours in the peritoneal space);
2. Measuring conductivity of the solution (in particular of the base conductivity $\Gamma_0$ of the patient at equilibrium, after the nocturnal dwell time or after a prolonged period in the peritoneal space);
3. Emptying the peritoneal cavity;
4. Flush-before-fill, in which a quantity of fresh peritoneal solution is transferred from the source S via the discharge line 2;

5. (Optional) measurement of the conductivity $\Gamma_C$ of the fresh peritoneal solution;

6. Loading the fresh peritoneal solution to fill the peritoneal cavity;

7. Discharging a quantity of peritoneal solution from the peritoneal cavity after a set dwell period (for example 60 minutes);

8. Measuring conductivity $\Gamma_{60}$ of the peritoneal solution found in the peritoneal cavity after a set dwell period (60 minutes);

9. Calculating the peritoneal dialysis parameters, in particular the functionality of the peritoneal membrane and the corresponding classification of conveying through the membrane; for example, it is possible to calculate:

$$\Delta\Gamma_{PET} = \Gamma_0 - \Gamma_{60}.$$

The classification can be as follows:

$\Delta\Gamma_{PET} > 0.45$ mS/cm $\rightarrow$ defective conveying;

$0.25$ mS/cm $< \Delta\Gamma_{PET} \leq 0.45$ mS/cm $\rightarrow$ normal conveying;

$\Delta\Gamma_{PET} < 0.25$ mS/cm $\rightarrow$ optimum conveying.

Second example (manual mode):

1. Closing all the circuit lines, for example of the disposable PD set, in particular by driving the corresponding closing means;

2. Connecting the circuit to the source of fresh peritoneal solution (loading bag), to the discharge (discharge bag) and to the patient (access device giving access to the peritoneal catheter);

3. Connecting the measuring unit (conductimeter) to the PD circuit (in particular connecting the conductivity probe in the housing thereof inside the measuring basin B);

4. Switching on the screen 10 of the measuring unit 5;

5. Opening the discharge line 2 and opening the access line 3 to the patient to enable the peritoneal solution to be discharged from the peritoneal space;

6. Making a quantity of solution flow from the peritoneal space back into the discharge (discharge bag);

7. When the solution enters the discharge, closing the discharge line 2 and the access line 3;

8. Waiting a set time (for example 15 seconds) to read base conductivity $\Gamma_0$;

9. Storing the value of the base conductivity $\Gamma_0$;

10. Opening the discharge line 2 and the access line 3 to empty the peritoneal cavity;

11. Closing the patient access line 3;

12. Opening the delivery line 4 to make a small quantity of fresh solution flow (for example by a preset quantity or for a preset time, for example 5 seconds);

13. Closing the discharge line 2 and the delivery line 4;

14. Waiting for a certain period of time (for example 15 seconds) to read the conductivity $\Gamma_C$ of the fresh solution;

15. Opening the access line 3 and the delivery line 4 to enable the peritoneal cavity to be filled with fresh solution;

16. After filling has been completed, close all the lines;

17. Waiting for a set period of time (for example 1 minute from the end of filling);

18. Opening the discharge line 2 and the access line 3;

19. Making a quantity (for example 50 ml or mg) of solution (mix of fresh solution and residual liquid) flow from the peritoneal cavity to the discharge;

20. Closing the discharge line 2 and the access line 3;

21. Waiting for a few seconds (for example 15/20 seconds) to read the conductivity $\Gamma_1$ of the liquid (mixture of fresh solution and residual liquid) after dwelling in the peritoneal cavity for the aforesaid period of time period of time (1 minute);

22. Waiting for a set period of time (for example 60 from the end of filling);

23. Opening the discharge line 2 and the access line 3;

24. Making a quantity of used peritoneal solution (for example 50 ml or mg) flow back from the peritoneal cavity to the discharge (discharge bag);

25. Closing the discharge line 2 e the access line 3;

26. Waiting for a few seconds (for example 15/20 seconds) to read the conductivity $\Gamma_{60}$ of the liquid used after dwelling in the peritoneal cavity for the aforesaid period of time (60 minutes);

27. Calculating the desired parameter/s.

In this case the calculation may comprise determining the functionality of the peritoneal membrane and the conveying classification, as seen above and/or calculating the liquid VR remaining in the peritoneal cavity;

$$VR = V_{INF} \frac{\Gamma_1 - \Gamma_C}{\Gamma_0 - \Gamma_1}$$

where:

Vinf is obtained from the difference in the measurements of the scales immediately after the flush-before-fill and immediately after loading of the solution;
$\Gamma_0$ is measuring base conductivity (performed during the unloading to equilibrium step);
$\Gamma_C$ is measuring conductivity of the fresh solution (immediately after the flush-before-fill);
$\Gamma_1$ is measuring conductivity of the solution (mixture of fresh solution and residual liquid) discharged immediately after filling.

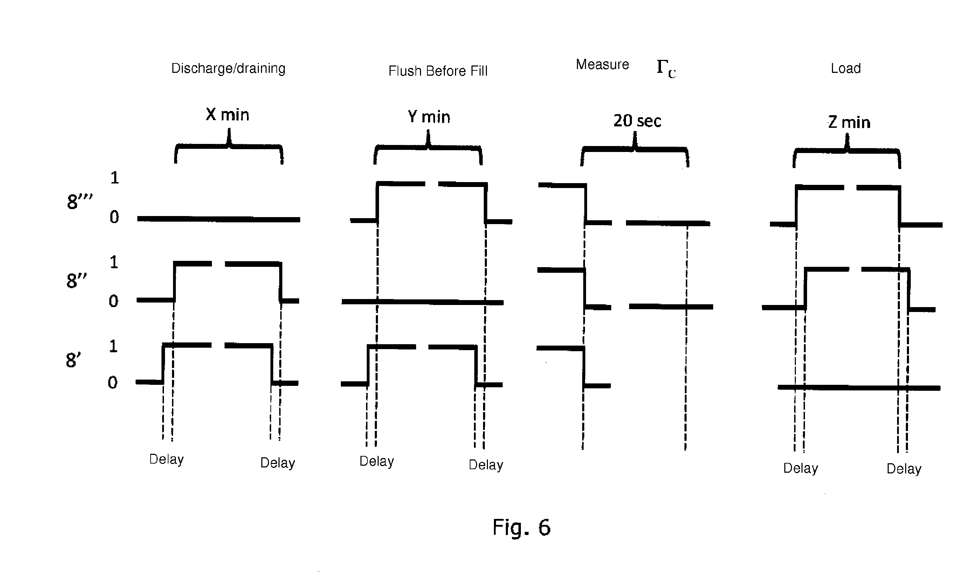

In figure 6 there is illustrated the time chart of the automatic control of the closing devices 8', 8", 8"' (similarly, the manual control of the clamps 6', 6", 6"' may be performed) in the case of determination of the conductivity of the fresh peritoneal solution. Numbers "1" and "0" indicate, respectively, the open state and the closed state of the devices. With X, Y and Z the time (in minutes) of the Discharge/Draining, Flush Before Fill and Loading steps are indicated.

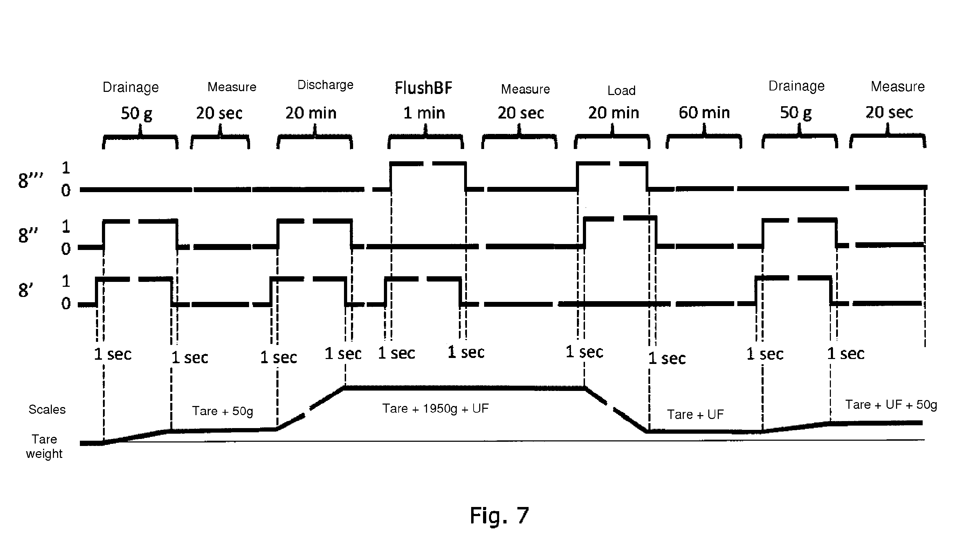

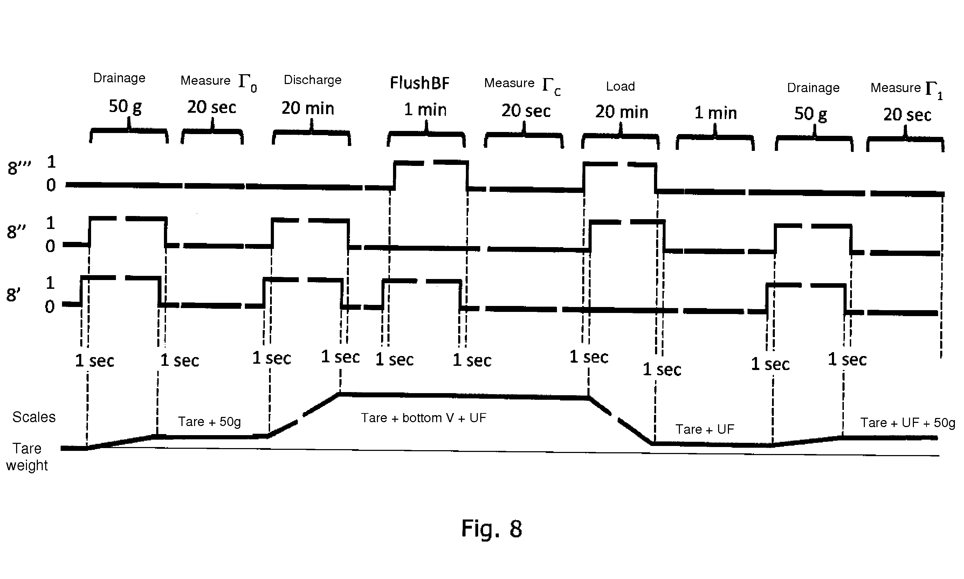

In each of figures 7 and 8 there is illustrated the time chart of the automatic control of the closing devices 8', 8", 8"' (similarly, the manual control of the clamps 6', 6", 6"' may be performed) in the case of measuring the conductivity, respectively, $\Gamma_0$, $\Gamma_C$ and $\Gamma_{60}$ (figure 7) and $\Gamma_0$, $\Gamma_C$ and $\Gamma_1$ (figure 8) that are usable to determine the functionality of the membrane and of the residual volume VR. The end of the partial drainage steps from the peritoneal space may be controlled through feedback by the weighing signal of the scales and a preset weight value (for example 50 grams).

With reference to what has been disclosed above, the instructions with which the control unit will be programmed comprise, in particular, the following operations: receiving at least one first value of electrical conductivity of a first liquid found in the extracorporeal circuit; receiving at least a second value of electrical conductivity of a second liquid (different from the first) found in the extracorporeal circuit; and determining at least one peritoneal dialysis parameter on the basis of a relation between the two conductivity values received. The relation may be defined on the basis, for example, of one of the mathematical models disclosed above.

The programme instructions may comprise the control of means for closing the circuit lines to permit: a discharge step, in which the first liquid is transferred from the peritoneal space to the extracorporeal circuit; a further first discharge step, or partial discharge step, in which the second liquid, after a dwell period in the peritoneal space that is different from the dwell period of the first liquid, is transferred, at least partially, from the peritoneal space to the extracorporeal circuit.

The programme instructions may comprise the control of means for closing the circuit lines to enable, after the discharge step in which a first electrical conductivity value of a first liquid found in the extracorporeal circuit is measured, the further steps of: rinsing before filling, in which the fresh peritoneal solution is transferred from the source S to the discharge D through the extracorporeal circuit; filling of the peritoneal space, in which the fresh solution is transferred from the source S to the peritoneal space; at least one first further discharge or partial discharge, in which, after a set dwell period in the peritoneal space, for example a period of about an hour from the end of the filling step, a second liquid is transferred from the peritoneal space to the extracorporeal circuit; measuring at least a second value of electrical conductivity of the second liquid found in the extracorporeal circuit; and determining at least one peritoneal dialysis parameter, in particular the functionality of the peritoneal membrane, on the basis of a relation between the first and the second measured conductivity value.

The programme instructions may comprise the control of means for closing the circuit lines to enable, after the discharge step in which a basic electrical conductivity value was measured of the liquid coming from the peritoneal cavity at the balance, after a prolonged dwell time, the further steps of: rinsing before filling, in which the fresh dialysis liquid is (partially) transferred from the source S of fresh solution to the discharge D through the extracorporeal circuit; measuring at least one value of the electrical conductivity of the fresh solution found in the extracorporeal circuit; filling of the peritoneal space, in which the fresh solution is transferred from the source S to the peritoneal space; at least a second further discharge, in which, after a second dwell period in the peritoneal space, for example a period of about a minute from the end of the filling step, a mixture of fresh solution and residual liquid is transferred from the peritoneal space to the extracorporeal circuit; measuring at least one electrical conductivity value of this mixture found in the extracorporeal circuit; and determining at least one peritoneal dialysis parameter, in particular the residual volume of used dialysis solution, on the basis of a relation between the measured conductivity values.

In the case of automated treatment, it is possible that the control logic unit, on the basis of the signal that the conductimeter (for example immersion conductimeter) arranged on the circuit (discharge line) and of the signal coming from a pair of scales (that weighs the loaded bag and the discharged bag), is able to pilot the three closing devices arranged on the circuit lines.

On the basis of the conductivity measurements it will be possible to determine the functionality of the peritoneal membrane and/or the residual volume of used dialysis solution that remains in the peritoneal space during the discharge step.

By processing the conductivity signals, it is also possible to determine the glucose concentrations of the fresh solutions.

The measuring unit 5 may have a system for digital reading and recording of data, for example on an electronic card, with the optional possibility of inserting each time the type of liquid that is being measured.

It is further possible that the control unit 9 is programmed for displaying on the screen pre-ordered step-by-step instructions. For example, after switching on of the measuring unit, a suggestion may appear (for example the "Drainage" button or icon), so the operator or the patient will be instructed to carry out the discharge and the unit 5 will measure the conductivity dialysate of the previous exchange; at this point, for example, by pressing a button (arrow or forwards) another message may appear (for example "Washing") and the operator/patient will be instructed to run flush before fill and the unit 5 will measure the conductivity of the fresh solution found in the loading bag; at this point the patient may be instructed to remain connected for a set time (for example 1 hour) and then discharge a small quantity (for example after an hour the control unit may command the issuing of a suggestion to perform a partial discharge such as a sound and the appearance of a message on the screen such as an icon "Partial discharge" or the like). It is possible, in a system provided with a flowmeter or similar measuring means, to emit a signal (for example a sound signal) after the discharge of the desired quantity of liquid (for example 50 ml or 50 g).

Algorithms may be further provided for running a peritoneal functionality (permeability) test at home. For example, from a menu that appears on the screen of the user interface, the "Peritoneal equilibration test " or "PET" may be selected with the instructions that appear on the screen, step-by-step, for performing the method in question to determine the functionality (permeability) of the peritoneal membrane.

The measuring unit could comprise, as said, an external conductimeter, which does not require direct contact; in this case the measuring unit may comprise a seat (for example a groove) that is able to receive in a removable connection, a line portion of the peritoneal dialysis circuit.

In one example, a monitor for APD (cycler for automated APD exchanges) may be integrated with the unit for measuring conductivity in such a manner that, for example, the unit for measuring conductivity reports to the monitor the residual volume VR and the monitor consequently controls the load volume during the break-point and/or controls the mixing proportion of two different glucose concentration solutions to have the desired concentration (which is optionally variable during treatment) in the peritoneal space. Further, in the case of an unexpected increase of the conductivity of the used dialysis solution, the emission of an alarm on the monitor may be provided that can, for example, indicate the need to conduct the Combur test and/or count white blood cells.

**Claims**

1.  Peritoneal dialysis system comprising:

    - an extracorporeal circuit that is connectable to a peritoneal access of a patient (P) for conveying liquid from/to a peritoneal space;
    - measuring means (5) for measuring at least one value of an electrical property of the liquid present in said extracorporeal circuit; and
    - means for determining at least one parameter on the basis of said at least one value, said at least one parameter comprising at least one of (i) functionality of the patient peritoneal membrane and (ii) residual volume of liquid remaining in the peritoneal space.

2.  System according to claim 1, wherein said means for determining determines said at least one parameter on the basis of at least one first value of the electrical property of a first liquid and at least one second value of the electrical property of a second liquid, the first liquid having spent a first dwell period in the peritoneal space and the second liquid having spent a second dwell period in the peritoneal space that is different from the first dwell period.

3.  System according to claim 2, wherein the first dwell period is a long period such that the electrical property of the first liquid is substantially the basic electrical property in a balance condition.

4. System according to claim 2 or 3, wherein said means for determining determines said at least one parameter on the basis of said at least one first value, said at least one second value and at least one third value of the electrical property of a fresh peritoneal solution in said extracorporeal circuit.

5. System according to claim 4, wherein said at least one third value is measured by said measuring means (5).

6. System according to any preceding claim, wherein said extracorporeal circuit has a discharge line (2) that is connectable to a discharge (D), an access line (3) for connecting the peritoneal access of the patient (P) to said discharge line, and a delivery line (4) for connecting a source (S) of fresh peritoneal solution to said discharge line and to said access line, said measuring means (5) being arranged in said discharge line (2).

7. System according to claim 6, comprising means for selectively closing said discharge line, access line and delivery line.

8. System according to any preceding claim, comprising:

- a programmable electronic control unit connected to said measuring means and to said means for determining;
- a medium which is readable by a computer and which has programme instructions that can be run by said control unit (9), wherein said programme instructions comprise:

* receiving at least one first value of the electrical property of a first liquid in said extracorporeal circuit;
* receiving at least one second value of the electrical property of a second liquid present in said extracorporeal circuit; and
* determining said at least one parameter on the basis of a relationship between said first and second value.

9. System according to claims 8 and 7, wherein said electronic control unit is arranged for controlling said closing means (8', 8", 8'''), and wherein said programme instructions comprise controlling said closing means in such a manner as to enable:

- the first liquid has a first dwell period in the peritoneal space, in particular a period long such that the electrical property of the first liquid is substantially the basic electrical property in a balance condition;
- the first liquid is transferred from the peritoneal space to said extracorporeal circuit during a discharge step;
- the second liquid has a second dwell period in the peritoneal space that is different from the first dwell period;
- the second liquid is transferred from the peritoneal space to said extracorporeal circuit during at least a first further discharge.

10. System according to claim 9, wherein said programme instructions comprise controlling said closing means to permit, after said discharge step, the steps of:

- rinsing before filling, wherein a fresh peritoneal solution is transferred from said source to said discharge through said extracorporeal circuit;
- filling the peritoneal space, wherein the fresh peritoneal solution is transferred from the source to the peritoneal space.

11. System according to claim 9 or 10, wherein said second dwell period in the peritoneal space is comprised between approximately 15/30 minutes and 90/120 minutes, for example approximately 60 minutes, from the end of said filling step.

12. System according to claim 9, wherein said programme instructions comprise controlling said closing means to enable, after said discharge step, the steps of:

- rinsing before filling, wherein a fresh peritoneal solution is transferred from said source to said discharge through said extracorporeal circuit;
- receiving at least a third value of the electric property of the fresh peritoneal solution present in said extracorporeal circuit;
- filling the peritoneal space, wherein the fresh peritoneal solution is transferred from the source to the peritoneal space, said second dwell period in the peritoneal space being in particular comprised between approximately 15/30 seconds and 90/120 seconds, for example about 60 seconds, from the end of said filling step, said at least one parameter being determined by a relationship between said first, second and third value.

13. System according to any preceding claim, comprising:

- a programmable electronic control unit connected to said measuring means and to said means for determining;
- a medium which is readable by a computer and which has programme instructions that can be run by said control unit (9), wherein said programme instructions comprise receiving said at least one value and determining said at least one parameter on the basis of said at least one value.

14. System according to any preceding claim, wherein said electrical property comprises electrical conductivity.

15. Method for determining at least one parameter during peritoneal dialysis, said at least one parameter comprising at least one of (i) functionality of the peritoneal membrane of the patient and (ii) residual volume of liquid remaining in the peritoneal space of the patient, wherein, during a peritoneal dialysis discharge step, a first liquid, which has spent a first dwell period in the peritoneal space, is transferred from the peritoneal space to an extracorporeal circuit for peritoneal dialysis, the method comprising the steps of measuring at least one first value of an electrical property of said first liquid in said extracorporeal circuit and determining said at least one parameter on the basis of said at least one first value; said method further comprising in particular:

- at least one optional step of further discharge wherein a second liquid, after spending a second dwell period in the peritoneal space that is different from the first dwell period, is transferred from the peritoneal space to said extracorporeal circuit;
- at least one optional step of measuring at least one second value of said electrical property of said second liquid present in said extracorporeal circuit;
- at least one optional step of determining said at least one parameter on the basis of a relationship between said first and second value;
- at least one optional step of rinsing before filling, wherein an amount of fresh peritoneal solution is transferred from a source of fresh solution to a discharge through said extracorporeal circuit;
- at least one optional step of filling of the peritoneal space, wherein the fresh peritoneal solution is transferred from the source to the peritoneal space;
- said second dwell period in the peritoneal space being in particular comprised between about 15/30 minutes and 90/120 minutes, for example about 60 minutes, from the end of said filling step, said first dwell period being a prolonged period in such a manner that the electrical property of the first liquid is substantially the basic electrical property in a state of equilibrium, said parameter being the functionality of the peritoneal membrane;
- at least one optional step of measuring at least one third value of the electrical property of the fresh peritoneal solution present in said extracorporeal circuit;
- said second dwell period in the peritoneal space being in particular comprised between about 15/30 seconds and 90/120 seconds, for example about 60 seconds, from the end of said filling step, said parameter being the residual volume of liquid remaining in the peritoneal space at the end of said discharge step determined on the basis of a relationship between said first, second and third value;
- said electrical property comprising in particular electrical conductivity.

Fig. 1

Fig. 2

EP 2 623 139 A1

Fig. 3

Fig. 5

13

3,86%

10

Drainage — 11

Load

Solution

PET — 11

Residual volume

Fig. 4

Fig. 6

Fig. 7

Fig. 8

Europäisches
Patentamt

European
Patent Office

Office européen
des brevets

## EUROPEAN SEARCH REPORT

**Application Number**

EP 13 15 2920

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 96/33753 A1 (BAXTER INT [US]) 31 October 1996 (1996-10-31) * page 4, line 11 - page 5, line 21 * * page 8, lines 6-12 * * figures 1,2 * | 1-5,8, 13,14 | INV. A61M1/28 |
| Y | WO 2011/005387 A1 (BAXTER INT [US]; BAXTER HEALTHCARE SA [CH]; LO YING-CHENG [US]; AKONUR) 13 January 2011 (2011-01-13) * paragraphs [0019], [0053] * | 1-5,8, 13,14 | |
| A | US 2009/007642 A1 (BUSBY DONALD [US] ET AL) 8 January 2009 (2009-01-08) * paragraph [0008] - paragraph [0010]; claims 1-22 * * paragraph [0040] - paragraph [0048] * * paragraph [0072] - paragraph [0078] * | 1-14 | |
| A | WO 2005/035023 A1 (GAMBRO LUNDIA AB [SE]; JANSSON OLOF [SE]; BERNARD PASCAL [FR]; DURAND) 21 April 2005 (2005-04-21) * page 14, lines 1-14 * * page 18, lines 20-29 * * figure 2 * | 1 | TECHNICAL FIELDS SEARCHED (IPC) A61M |
| A | WO 03/063929 A1 (DEBIOTECH SA [CH]; NEFTEL FREDERIC [CH]) 7 August 2003 (2003-08-07) * page 7, lines 13-34 * * figures 1,2 * | 1,6,7,10 | |

The present search report has been drawn up for all claims

| Place of search | Date of completion of the search | Examiner |
|---|---|---|
| Munich | 16 May 2013 | Bichlmayer, K |

**ANNEX TO THE EUROPEAN SEARCH REPORT
ON EUROPEAN PATENT APPLICATION NO.**  EP 13 15 2920

This annex lists the patent family members relating to the patent documents cited in the above-mentioned European search report.
The members are as contained in the European Patent Office EDP file on
The European Patent Office is in no way liable for these particulars which are merely given for the purpose of information.

16-05-2013

| Patent document cited in search report | | Publication date | Patent family member(s) | | Publication date |
|---|---|---|---|---|---|
| WO 9633753 | A1 | 31-10-1996 | AT | 253950 T | 15-11-2003 |
| | | | DE | 69630660 D1 | 18-12-2003 |
| | | | DE | 69630660 T2 | 07-10-2004 |
| | | | EP | 0782460 A1 | 09-07-1997 |
| | | | JP | 3895770 B2 | 22-03-2007 |
| | | | JP | H10502569 A | 10-03-1998 |
| | | | US | 5670057 A | 23-09-1997 |
| | | | WO | 9633753 A1 | 31-10-1996 |
| WO 2011005387 | A1 | 13-01-2011 | EP | 2451500 A1 | 16-05-2012 |
| | | | JP | 2012532669 A | 20-12-2012 |
| | | | US | 2011010101 A1 | 13-01-2011 |
| | | | WO | 2011005387 A1 | 13-01-2011 |
| US 2009007642 | A1 | 08-01-2009 | US | 2009007642 A1 | 08-01-2009 |
| | | | WO | 2009009227 A2 | 15-01-2009 |
| WO 2005035023 | A1 | 21-04-2005 | AT | 386557 T | 15-03-2008 |
| | | | AU | 2004279280 A1 | 21-04-2005 |
| | | | CA | 2536421 A1 | 21-04-2005 |
| | | | DE | 602004011981 T2 | 05-03-2009 |
| | | | EP | 1691863 A1 | 23-08-2006 |
| | | | ES | 2298818 T3 | 16-05-2008 |
| | | | JP | 4658062 B2 | 23-03-2011 |
| | | | JP | 2007508101 A | 05-04-2007 |
| | | | KR | 20060128862 A | 14-12-2006 |
| | | | WO | 2005035023 A1 | 21-04-2005 |
| WO 03063929 | A1 | 07-08-2003 | AT | 479456 T | 15-09-2010 |
| | | | EP | 1469896 A1 | 27-10-2004 |
| | | | JP | 4303125 B2 | 29-07-2009 |
| | | | JP | 2005515845 A | 02-06-2005 |
| | | | US | 2005089994 A1 | 28-04-2005 |
| | | | US | 2012310056 A1 | 06-12-2012 |
| | | | WO | 03063929 A1 | 07-08-2003 |

For more details about this annex : see Official Journal of the European Patent Office, No. 12/82